# EUROPEAN PATENT APPLICATION

(11) **EP 3 193 239 A1**
(43) Date of publication of application: **19.07.2017**
(21) Application number: 17151183.5
(22) Date of filing: 12.01.2017
(51) Int. Cl.: G06F 3/01, G06F 19/00, A61F 4/00

(54) **METHODS AND SYSTEMS FOR AUGMENTATIVE AND ALTERNATIVE COMMUNICATION**

(30) Priority: 12.01.2016 US 201614993889
(71) Applicant: University Of Iowa Research Foundation, Iowa City, IA (US); Bird, Richard Rives, Coralville, IA 52241 (US)
(72) Inventor: Bird, Richard Rives, Coralville, IA Iowa 52241 (US); Hurtig, Richard, Philadelphia, PA Pennsylvania 19106 (US); Berkowitz, Benjamin, Iowa City, IA Iowa 52246 (US); Martinson, Blake Earl, Charlotte, NC North Carolina 28203 (US); Zhu, Zihan, Iowa City, IA Iowa 52246 (US)
(74) Representative: CH Kilger Anwaltspartnerschaft mbB

(57) **Abstract**

Methods and systems for augmentative and alternative communication are disclosed. An example method can comprise receiving a candidate input, classifying the candidate input as an intentional input, and generating a signal in response to the intentional input.

## Description

### BACKGROUND

Current hospital accreditation standards mandate that all patients be provided with the means to summon the assistance of caregivers (e.g. nurse call). Many critical care patients are too weak to activate standard or alternative nurse call switches that are available at most facilities. Mechanical ventilation further limits patients to use voice to summon or communicate with caregivers. For many patients in intensive care and long term acute care settings, there are no existing communication methods that can transduce the minimal intentional gestures that these patients are capable of making. These and other shortcomings are addressed in the present disclosure.

### SUMMARY

It is to be understood that both the following general description and the following detailed description are exemplary and explanatory only and are not restrictive, as claimed. Provided are methods and systems for augmentative and alternative communication. An example method can comprise receiving a candidate input, classifying the candidate input as an intentional input, and generating a signal in response to the intentional input.

Another example method can comprise receiving a candidate input. The candidate input can be classified as an intentional input. One or more devices associated with the intentional input can be determined. A signal can be generated to control the one or more devices associated with the intentional input.

An example apparatus can comprise an input interface, an output interface, a memory and a processor coupled to the input interface, the output interface, and the memory. The input interface can be configured for receiving a candidate input from one or more sensors. The output interface can be configured for transmitting a signal to a controlled device. The memory can be configured for storing a filter. The processor can be configured to classify the candidate input received by the input interface by applying the filter stored in the memory, and providing the signal to the output interface for transmission to the controlled device.

Additional advantages will be set forth in part in the description which follows or may be learned by practice. The advantages will be realized and attained by means of the elements and combinations particularly pointed out in the appended claims.

### BRIEF DESCRIPTION OF THE DRAWINGS

The accompanying drawings, which are incorporated in and constitute a part of this specification, illustrate embodiments and together with the description, serve to explain the principles of the methods and systems:
Figure 1 is an exemplary apparatus capable of performing the methods disclosed;
Figure 2A illustrates average spectrum of a tongue click in frequency domain;
Figure 2B illustrates signal intensity of tongue click in time domain;
Figure 2C illustrates average spectrum of fan noise and tongue clicks in frequency domain;
Figure 2D illustrates signal intensity of fan noise and tongue clicks in time domain;
Figure 2E illustrates average spectrum of a filtered tongue click and fan noise in frequency domain;
Figure 2F illustrates signal intensity of a filtered tongue click and fan noise in time domain;
Figure 3 is block diagram illustrating an audio signal detection method;
Figure 4A illustrates an example apparatus using analog filtering;
Figure 4B illustrates an example apparatus using digital filtering;
Figure 5 illustrates the logic of a signal detection algorithm;
Figure 6 is a flow chart illustrating an exemplary method;
Figure 7 is a flow chart illustrating an exemplary method;
Figure 8 is exemplary apparatus;
Figure 9 is exemplary system;
Figure 10 is exemplary operating environment;
Figure 11A shows a top-down perspective view of an example apparatus and a clamp;
Figure 11B shows a bottom-up perspective view of the example apparatus and the clamp;
Figure 11C shows a side-view of the example apparatus and the clamp;
Figure 11D shows another side-view of the example apparatus and the clamp;
Figure 12A shows an example sensor mounting;
Figure 12B shows an another example sensor mounting;
Figure 12C shows an another example sensor mounting;
Figure 12D shows an another example sensor mounting;
Figure 13A illustrates an example apparatus with an pole mounted speech generating device;
Figure 13B illustrates an example user interface provided by the display;
Figure 13C shows another view of the mounting device and display device;
Figure 14 is a block diagram illustrating an example apparatus;
Figure 15 is a block diagram illustrating another example apparatus;
Figure 16 is a block diagram of an apparatus illustrating aspects of an example sensor recognition switch;
Figure 17 illustrates an example processing unit of the apparatus;
Figure 18 illustrates an example power management system;
Figure 19 is a flow chart illustrating an example method for managing a device;
Figure 20 is a flow chart illustrating an example method for recognizing a gesture; and
Figure 21 is a flow chart illustrating an example method for recognizing a gesture.

### DETAILED DESCRIPTION

Before the present methods and systems are disclosed and described, it is to be understood that the methods and systems are not limited to specific methods, specific components, or to particular implementations. It is also to be understood that the terminology used herein is for the purpose of describing particular embodiments only and is not intended to be limiting.

As used in the specification and the appended claims, the singular forms "a," "an" and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

"Optional" or "optionally" means that the subsequently described event or circumstance may or may not occur, and that the description includes instances where said event or circumstance occurs and instances where it does not.

Throughout the description and claims of this specification, the word "comprise" and variations of the word, such as "comprising" and "comprises," means "including but not limited to," and is not intended to exclude, for example, other components, integers or steps. "Exemplary" means "an example of" and is not intended to convey an indication of a preferred or ideal embodiment. "Such as" is not used in a restrictive sense, but for explanatory purposes.

Disclosed are components that can be used to perform the disclosed methods and systems. These and other components are disclosed herein, and it is understood that when combinations, subsets, interactions, groups, etc. of these components are disclosed that while specific reference of each various individual and collective combinations and permutation of these may not be explicitly disclosed, each is specifically contemplated and described herein, for all methods and systems. This applies to all aspects of this application including, but not limited to, steps in disclosed methods. Thus, if there are a variety of additional steps that can be performed it is understood that each of these additional steps can be performed with any specific embodiment or combination of embodiments of the disclosed methods.

The present methods and systems may be understood more readily by reference to the following detailed description of preferred embodiments and the examples included therein and to the Figures and their previous and following description.

As will be appreciated by one skilled in the art, the methods and systems may take the form of an entirely hardware embodiment, an entirely software embodiment, or an embodiment combining software and hardware aspects. Furthermore, the methods and systems may take the form of a computer program product on a computer-readable storage medium having computer-readable program instructions (e.g., computer software) embodied in the storage medium. More particularly, the present methods and systems may take the form of web-implemented computer software. Any suitable computer-readable storage medium may be utilized including hard disks, CD-ROMs, optical storage devices, or magnetic storage devices.

Embodiments of the methods and systems are described below with reference to block diagrams and flowchart illustrations of methods, systems, apparatuses and computer program products. It will be understood that each block of the block diagrams and flowchart illustrations, and combinations of blocks in the block diagrams and flowchart illustrations, respectively, can be implemented by computer program instructions. These computer program instructions may be loaded onto a general purpose computer,-special purpose computer, or other programmable data processing apparatus to produce a machine, such that the instructions which execute on the computer or other programmable data processing apparatus create a means for implementing the functions specified in the flowchart block or blocks.

These computer program instructions may also be stored in a computer-readable memory that can direct a computer or other programmable data processing apparatus to function in a particular manner, such that the instructions stored in the computer-readable memory produce an article of manufacture including computer-readable instructions for implementing the function specified in the flowchart block or blocks. The computer program instructions may also be loaded onto a computer or other programmable data processing apparatus to cause a series of operational steps to be performed on the computer or other programmable apparatus to produce a computer-implemented process such that the instructions that execute on the computer or other programmable apparatus provide steps for implementing the functions specified in the flowchart block or blocks.

Accordingly, blocks of the block diagrams and flowchart illustrations support combinations of means for performing the specified functions, combinations of steps for performing the specified functions and program instruction means for performing the specified functions. It will also be understood that each block of the block diagrams and flowchart illustrations, and combinations of blocks in the block diagrams and flowchart illustrations, can be implemented by special purpose hardware-based computer systems that perform the specified functions or steps, or combinations of special purpose hardware and computer instructions.

Provided are methods and systems for augmentative and alternative communication. The disclosed methods and systems can process complex signals embedded in complex noise in order to use the signals to allow a patient or user to control one or more devices in their environment, e.g. activating nurse call or patient controlled pain pumps, speech generating communication devices, and the like. In an aspect, the disclosed methods and systems can improve signal interpretation for a diverse set of input devices (e.g., acoustic, pressure, proximity and reflectance) used for augmentative and alternative communication devices for a variety of users. An exemplary application can include patients in a hospital setting who are paralyzed or severely incapacitated. The methods and systems provided can eliminate background noise (e.g., from hospital equipment) and other environmental sources as well as non-communication initiated movements from the patient (for example, normal eye blinks). The disclosed methods and systems can distinguish the intentional gesture from the ambient noise received from the environment or from non-intentional tremor in the patient's gestures. In an aspect, the methods and systems disclosed can be used in a hospital environment to enable intubated and otherwise disabled patients to communicate with caregivers. The methods and systems can be used by other care facilities such as outpatient facilities and elderly care facilities.

In an aspect, the disclosed methods and systems can comprise signal processing that can analyze the kinematics of intentional gestures that can be transduced and serve as inputs. For example, the methods can comprise intentional input detection regardless of the input modality (e.g., audio, tactile, pressure, IR reflectance). For example, rather than responding each time a user touches a switch plate, a signal processing switch can effectively filter out the unintentional touches and only respond to an intentional touch. This can be achieved by identifying the intentional gesture based on characteristics of the intentional gesture in the frequency domain, the time domain, or both.

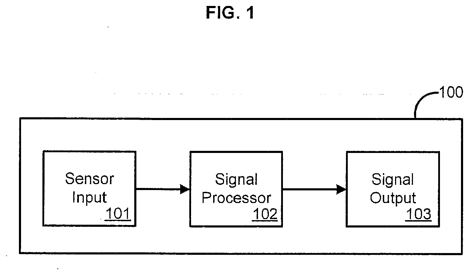

An exemplary apparatus **100,** shown in **FIG. 1****,** can comprise one or more sensor inputs **101** for sensors such as microphones, pressure bulbs, pressure transducers, and the like. These sensor inputs can be processed by a signal processor **102** to classify a specific stimulus as intentional, as distinguished from noise and unintentional stimuli. The apparatus can be configured to a signal output **103** to control a device, which can be activated by the intentional stimulus and an optional visual output which can display to the user a menu of instructions and options. As an example, a controlled device can comprise one or more of a nurse call switch, a computer, a television, a telephone, a medical device, an application (e.g., iPad applications), a speech generating device, and the like. In an aspect, the signal output **103** can be used to invoke keyboard commands for communication or controlling other mechanical or computerized outputs. In an aspect, any type of sensor and any type of output can be utilized.

In an aspect, the methods and systems can be configured to operate in conjunction with a touch switch. A common problem with signals from switches activated by touch (either mechanical or capacitance/proximity detector switches) is that in order to respond to gestures that generate minimal displacement and force, the sensors are so sensitive that they are often activated unintentionally by tremors that often accompany the intentional gesture. A characteristic of the tremor can be determined and processed to generate a filter. For example, a tremor can generate a signal from about 4Hz to about 12Hz, for example a 4Hz, 5Hz, 6Hz, 7Hz, 8Hz, 9Hz, 10Hz, 11Hz, and/or 12Hz signal. The disclosed methods and systems can comprise applying a low pass filter to the signal received from a touch switch to account for the tremors. In an aspect, an intentional tremor can have a frequency of below about 5Hz. The methods and systems can then respond to the underlying intentional gesture and not to any higher frequency signals received from the transducer that are the consequence of the tremor in the gesture.

In an aspect, the methods and systems can be configured to operate in conjunction with a pressure sensor. For patients who cannot generate either the force or displacement necessary to activate touch switches, a pressure sensor can be used. Pressure sensors can be implemented as "sip-and-puff switches" used by individuals who are quadriplegic or paraplegic. These switches require that the user generate adequate changes in oral pressure. For individuals who cannot generate the oral pressure needed to activate the "sip-and-puff switches," one can use a closed silastic bulb that only requires a lip closure or a biting gesture. Such a bulb can be positioned just to the side of the user's mouth and a small lip gesture can allow the user to grasp the bulb and thus generate a positive pressure change. However, many intubated or trached patients, who are also unable to move, prefer to keep the silastic bulb in their mouths. A consequence of this is that the small inadvertent movements of the lips and tongue that are not associated with the intentional gesture are also generating pressure changes. The disclosed methods and systems can determine a characteristic of the inadvertent movements and generate a filter to account for the inadvertent movements. For example, the methods and systems can analyze a time course of lip gestures and magnitude of the pressure changes associated with an intentional gesture, thereby generating a filter that can discriminate between intentional and unintentional inputs based on particular pressure changes over a particular time interval.

In an aspect, the methods and systems can be configured to operate in conjunction with an infrared sensor, such as an infrared reflectance switch. For many spinal cord injury patients as well as patients with locked-in-syndrome, the only motor gestures that they can reliably generate involve eyelid control. An eye-blink switch can be utilized to detect changes in reflected infrared light that are a consequence of eyelid movements. A problem is that existing eye-blink switches have difficulty distinguishing the reflectance changes associated with an intentional blink gesture from those associated with unintentional blink gestures, such as natural blinks or slower lid movements associated with either gaze shifts or lid dropping associated with drowsiness. The disclosed methods and systems can determine a characteristic of the intentional movements and generate a filter to account for the unintentional movements. In an aspect, the disclosed methods and systems can use kinematic analysis of eyelid movements for blink detection which is not responsive to the rapid reversal of reflectance associated with a natural blink, nor to the slower reflectance changes associated with slower involuntary lid movements. The methods and systems can set temporal cutoffs that only respond to the reflectance changes associated with the intermediate temporal duration of the intentional blink/wink.

In an aspect, the methods and systems can be configured to operate in conjunction with an audio sensor. For individuals who are able to generate sound, a Schmidt-trigger circuit (e.g., Voice Switch, Clapper switch) can be used. A problem with such a switch is that the switch can be triggered by a wide range of sounds and are not responsive to just a particular sound. As a result, there are many false positives and false negatives that result from variations in the audio signal intensity. In a hospital environment, many patients attempt to get staff attention by generating "small mouth" sounds. These sounds are made by small tongue movements or by popping of the lips. These are acoustically very low-amplitude signals that are often buried in the ambient noise. Ambient noise can include, for example, noise generated by equipment in the intensive care setting or by respiratory or gustatory sounds generated by the patient.

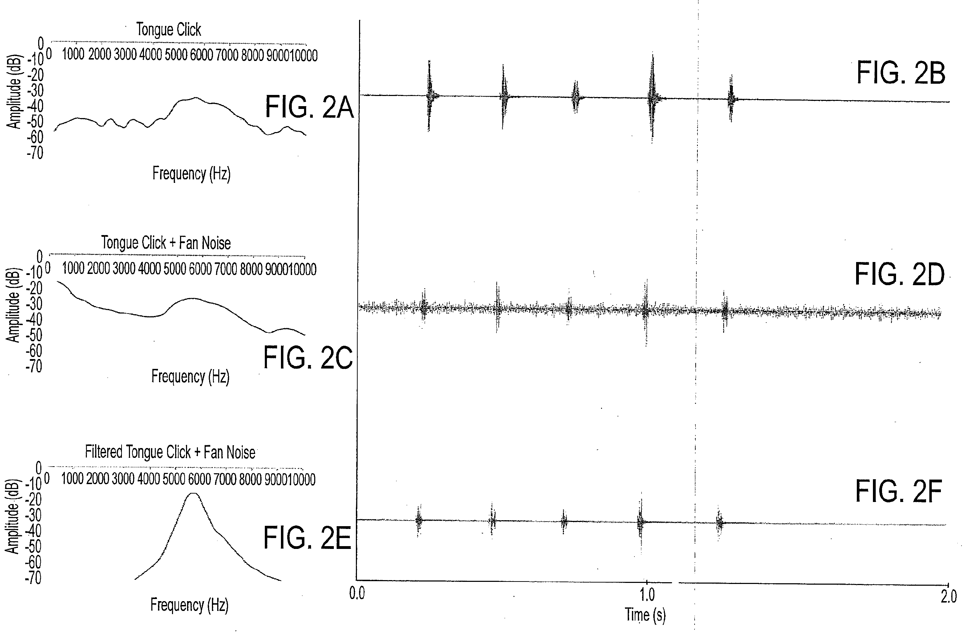

The disclosed methods and systems can determine a characteristic of the intentional movements and generate a filter to account for the unintentional movements. For example, the methods and systems can employ an acoustic analysis of intentional sounds (e.g., the "small mouth" sounds) to determine a time domain and frequency domain signature. The disclosed methods and systems can apply a filter (e.g., digital filter, analog filter) to an audio signal picked up by an audio sensor in order to identify intentional inputs. By way of example, **FIGS. 2A-2F** illustrate signal and spectral characteristics of environmental noise and a target mouth sound. Specifically, **FIG. 2A** illustrates average spectrum of a tongue click in frequency domain. **FIG. 2B** illustrates signal intensity of tongue click in time domain. **FIG. 2C** illustrates average spectrum of fan noise and tongue clicks in frequency domain. **FIG. 2D** illustrates signal intensity of fan noise and tongue clicks in time domain. **FIG. 2E** illustrates average spectrum of a filtered tongue click and fan noise in frequency domain. **FIG. 2F** illustrates signal intensity of a filtered tongue click and fan noise in time domain. As shown in **FIG. 2C** and **FIG. 2D****,** an intended tongue click is obscured by a background noise that is not desired, for example, fan noise. The signals representing a tongue click and a fan noise added together can be an input to a band-pass filter that would pass the characteristic frequencies of an intended tongue click. The output signal of the band-pass filter is shown in **FIG. 2E** and **FIG. 2F****.** By defining a filter that can pass a desired predetermined frequency range, unintended false positive signal detections can be prevented.

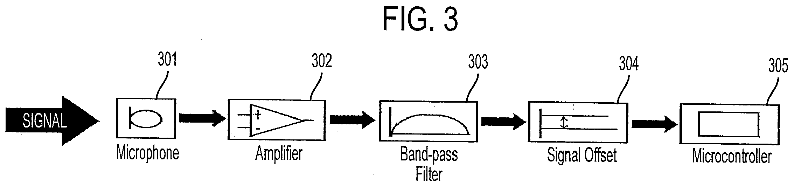

**FIG. 3** is a block diagram of an exemplary audio signal detection algorithm. At block **301,** an input signal can be received by an audio sensor, such as a microphone. At block **302,** the input signal can be amplified and passed through a band-pass filter at block **303.** At block **304,** the filtered input signal can be offset and provided to a microcontroller at block **305.** The audio input signal passes from the microphone to an amplifier and the band-pass filter whose cutoff frequencies are set to limit the signal to the frequency range of a targeted signal. The offset block **304** can be used since many signal processing chips operate only with positive voltages. The micro-controller can determine whether the input signal meets a characteristic (e.g., temporal characteristic) of an intended target signal and/or whether the input signal included one or more of the targeted signals.

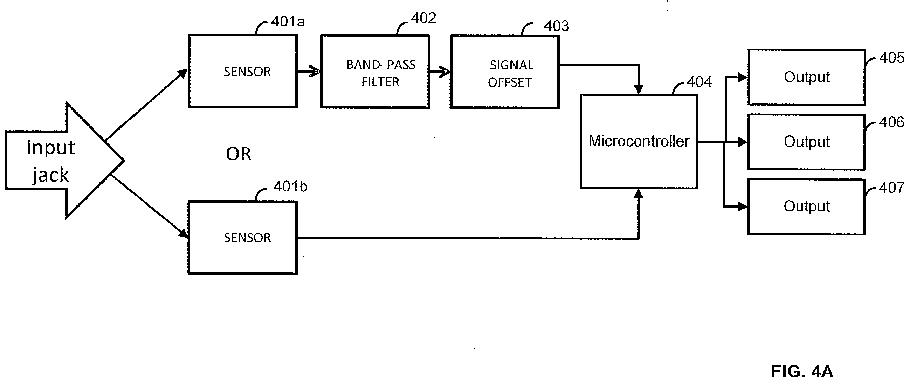

In an aspect, illustrated in **FIG. 4A****,** provided an example apparatus using analog filtering. One or more sensors (e.g., sensor **401a**) can be configured to receive one or more input signals via an input jack. For example, the modality of the one or more signals can comprise audio, tactile, pressure, IR reflectance, and the like. The one or more input signals can be applied to a filter (e.g., band-pass filter) **402.** For example, rather than responding each time a user touches a switch plate, the band-pass filter **402** can effectively filter out the unintentional touches and only respond to an intentional touch. In an aspect, the filter **402** can be generated by identifying an intentional gesture based on characteristics of the intentional gesture in the frequency domain, the time domain, or both. An offset can be applied to the filter input signal at block **403.** The processed input signal can be transmitted to a microcontroller **404.** In another aspect, an input signal does not need to be processed by the band-pass filter **402** and/or offset sensor **403.** For example, input signal can be received by sensor **401b** and transmitted to the microcontroller **404** directly (e.g., via an unfiltered channel). In an aspect, the microcontroller **404** can comprise one or more gesture detectors. In an aspect, the microcontroller **404** can not only detect the presence of an intentional input but can also track a number of inputs (intentional and/or unintentional) produced in succession. For example, one input signal (such as a first tap, a first blink, a first pressure bulb squeeze, or a first small mouth sound) can be used to activate a nurse call, while two input signals (e.g., such as a second tap, a second blink, a second pressure bulb squeeze, or a second small mouth sound) can be used to control an additional device (such as a fan or light), and three input signals (e.g., such as a third tap, a third blink, a third pressure bulb squeeze, or a third small mouth sound) can control yet another one or more devices (such as a TV). This allows a user who can only produce a single gesture to directly control a number of devices. As an example, a first intentional input can be sent to the output interface **405** for control a first device (e.g., nurse call). A second intentional input can be sent to the output interface **406** for control a second device (e.g., TV). A third intentional input can be sent to the output interface **407** for control a third device (e.g., computer). In an aspect, any number of output interfaces can be configured to control any number of devices.

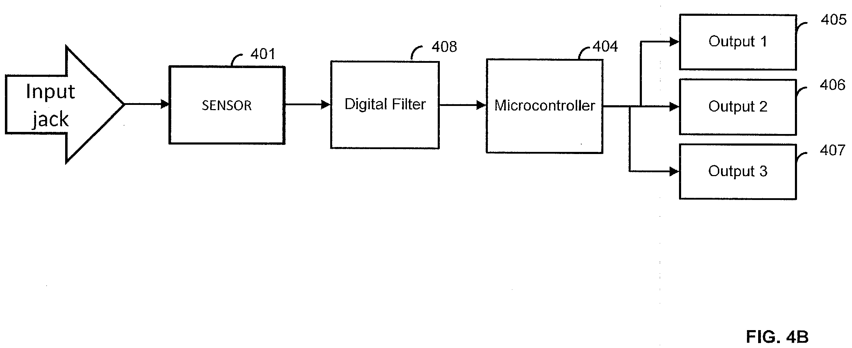

In an aspect, illustrated in **FIG. 4B****,** provided an example apparatus using digital filtering. As an example, a digital filter **408** can be applied to the input signals received via sensor **401.** The digitally filtered input signals can be transmitted to the microcontroller **404.** In an aspect, the microcontroller **404** can comprise one or more gesture detectors. In an aspect, the microcontroller **404** can not only detect the presence of an intentional input but can also track a number of inputs (intentional and/or unintentional) produced in succession. In an aspect, a first intentional input can be sent to the output interface **405** for control a first device (e.g., nurse call). A second intentional input can be sent to the output interface **406** for control a second device (e.g., TV). A third intentional input can be sent to the output interface **407** for control a third device (e.g., computer).

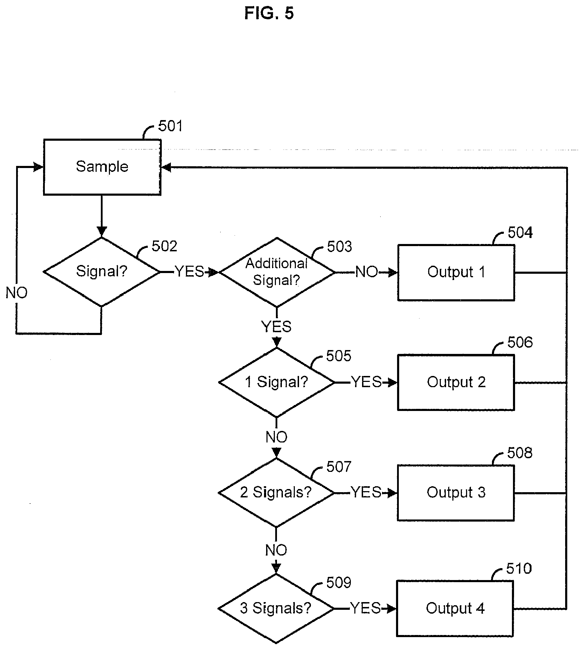

**FIG. 5** illustrates the logic of a signal detection algorithm. At block **501,** an input interface can be sampled to determine if a signal exists. If it is determined at block **502** that no signal exists, then methods can return to block **501.** If a signal exists, the methods can proceed to block **503** to determine if a subsequent signal exists. If no subsequent signal exists, the methods can generate an output at block **504** and return to block **501.** If an additional signal exists, it can be determined whether only one additional signal exists at block **505.** If only one additional signal exists, the methods can generate an output at block **506** and return to block **501.** If an additional signal exists, it can be determined whether only two additional signals exist at block **507.** If only two additional signals exist, the methods can generate an output at block **506** and return to block **501.** If an additional signal exists, it can be determined whether only three additional signals exist at block **509.** If only three additional signals exist, the methods can generate an output at block **510** and return to block **501.** A variety of inputs can be utilized and a variety of outputs can be generated at each signal processing step.

The present methods and systems can be operational with numerous other general purpose or special purpose computing system environments or configurations. Examples of well known computing systems, environments, and/or configurations that can be suitable for use with the systems and methods comprise, but are not limited to, personal computers, server computers, laptop devices, and multiprocessor systems. Additional examples comprise set top boxes, programmable consumer electronics, network PCs, minicomputers, mainframe computers, distributed computing environments that comprise any of the above systems or devices, and the like.

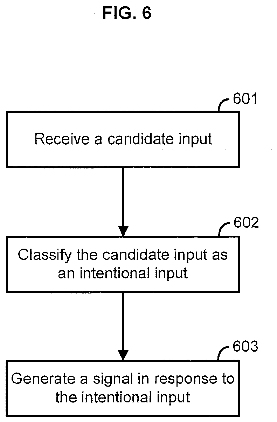

In an aspect, illustrated in **FIG. 6****,** provided are methods for communication. At block **601,** a candidate input can be received. In an aspect, a candidate input can be received from one or more sensors. As an example, the one or more sensors can comprise one or more of a touch sensor, a pressure sensor (e.g., oral pressure sensor), an optical reflectance sensor (e.g., infrared reflectance sensor), an electromyography (EMG) sensor, a light sensor, a proximity detector, an audio sensor, a mechanical sensor, a capacitance sensor, a camera, and the like. In an aspect, any type of sensor and any type of output can be utilized. In an aspect, a candidate input can be received from binary switches such as push buttons, capacitive touches, squeeze switches, and the like.

At block 602, the candidate input can be classified as an intentional input. In an aspect, classifying the candidate input as an intentional input can comprise determining a characteristic of an intentional input, generating a filter based on the characteristic, and applying the filter to the candidate input. In an aspect, the characteristic can be derived from a frequency domain, a time domain, or both. Such time-domain and frequency-domain characteristics can comprise properties such as a time domain intensity envelope and/or a frequency domain spectral shape. As an example, the candidate input can be processed by a signal processor **102** to classify a specific stimulus as intentional, as distinguished from noise and unintentional stimuli.

As an example, a characteristic of a tremor can be determined and processed to generate a filter. For example, a tremor can generate a signal from about 4Hz to about 12Hz, for example a 4Hz, 5Hz, 6Hz, 7Hz, 8Hz, 9Hz, 10Hz, 11Hz, and/or 12Hz signal. An intentional tremor can have a frequency of below about 5Hz. A low pass filter can be applied to the signal received from a touch switch to account for the tremors.

As an example, a characteristic of a pressure can be determined and processed to generate a filter. For example, the time course of an intentional lip closing gesture and magnitude of the pressure changes associated with the intentional lip closing gesture can be analyzed. For example, the intentional biting down on the bulb will generate a significantly larger pressure change than the inadvertent rolling of the tongue over the bulb. A filter can be generated to discriminate between intentional and unintentional inputs based on particular pressure changes over a particular time interval. For example, a temporal filter can distinguish the rapid changes in pressure associated with an intentional oral gesture from the slower changes in pressure associated with a non-intentional oral gesture. The temporal filter can also distinguish larger magnitude pressure changes from the smaller pressure changes that may be associated with accidental compression of a sylastic bulb used to detect oral movements.

As another example, a characteristic of an eyelid movement can be determined and processed to generate a filter. For example, eye-blink switches can be utilized that detects changes in reflected infrared light that are a consequence of eyelid movements. In an aspect, kinematic analysis of eyelid movements for blink detection can be used to set temporal cutoffs that only respond to the reflectance changes associated with the intermediate temporal duration of the intentional blink/wink.

As another example, a characteristic of a sound can be determined and processed to generate a filter. A filter can be generated to discriminate between the sounds made by small tongue movements and ambient noise. Two modes of filtering can be used, specifically, a filter in the frequency domain and a filter in the time domain. For example, a candidate signal can be first passed through a band-pass filter to eliminate candidate signals that are not in a known frequency range of the intentional gesture sound. However, other environmental noises may occur in similar frequency ranges as the intentional gesture sounds, accordingly, a temporal filtering can also be applied. For example, background noises can be of random temporal durations, while the intentional gesture sounds can be of known temporal durations. Therefore, by ignoring candidate signals that fall within a desired frequency range of the intentional gesture sounds and distinguishing temporal durations longer or shorter than a predetermined duration associated with an intentional gesture sound, the amount of false positive intentional inputs can be further reduced.

At block **603,** a signal can be generated in response to the intentional input. In an aspect, generating a signal in response to the intentional input can comprise activating a nurse call switch. In another aspect, generating a signal in response to the intentional input can comprise controlling one or more controlled devices. A controlled device can comprise one or more of a nurse call switch, a computer, a television, a telephone, a medical device, an application (e.g., iPad applications), a speech generating device, and the like. In an aspect, the generated signal can be used as keyboard commands for communication or controlling other mechanical or computerized outputs. In an aspect, the signal can comprise an optional visual output. The visual output can be displayed as a menu of instructions and options.

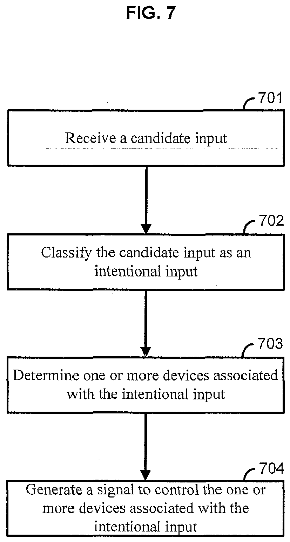

In an aspect, illustrated in **FIG. 7****,** provided are methods for communication. At block **701,** a candidate input can be received. In an aspect, a candidate input can be received from one or more sensors. As an example, the one or more sensors can comprise one or more of a touch sensor, a pressure sensor (e.g., oral pressure sensor), an optical reflectance sensor (e.g., infrared reflectance sensor), an electromyography (EMG) sensor, a light sensor, a proximity detector, an audio sensor, a mechanical sensor, a capacitance sensor, a camera, and the like. In an aspect, any type of sensor and any type of output can be utilized.

At block 702, the candidate input can be classified as an intentional input. In an aspect, classifying the candidate input as an intentional input can comprise determining a characteristic of an intentional input, generating a filter based on the characteristic, and applying the filter to the candidate input. In an aspect, the characteristic can be derived from a frequency domain, a time domain, or both. Such time-domain and frequency-domain characteristics can comprise properties such as a time domain intensity envelope and/or a frequency domain spectral shape.

In an aspect, an intentional input can comprise one or more signals. For example, the intentional input can comprise one or more gestures (e.g., eye blinks) with certain characteristics. As another example, the intentional input can comprise a sequence of gestures (e.g., eye blink followed by a tap). The intentional input can be used to control one or more devices. As an example, the candidate input can be processed by a signal processor **102** to classify a specific stimulus as intentional, as distinguished from noise and unintentional stimuli.

As an example, a characteristic of a tremor can be determined and processed to generate a filter. For example, a tremor can generate a signal from about 4Hz to about 12Hz, for example a 4Hz, 5Hz, 6Hz, 7Hz, 8Hz, 9Hz, 10Hz, 11Hz, and/or 12Hz signal. An intentional tremor can have a frequency of below about 5Hz. A low pass filter can be applied to the signal received from a touch switch to account for the tremors.

As an example, a characteristic of a pressure can be determined and processed to generate a filter. For example, the time course of an intentional lip closing gesture and magnitude of the pressure changes associated with the intentional lip closing gesture can be analyzed. For example, the intentional biting down on the bulb will generate a significantly larger pressure change than the inadvertent rolling of the tongue over the bulb. A filter can be generated to discriminate between intentional and unintentional inputs based on particular pressure changes over a particular time interval. For example, a temporal filter can distinguish the rapid changes in pressure associated with an intentional oral gesture from the slower changes in pressure associated with a non-intentional oral gesture. The temporal filter can also distinguish larger magnitude pressure changes from the smaller pressure changes that may be associated with accidental compression of a sylastic bulb used to detect oral movements.

As another example, a characteristic of an eyelid movement can be determined and processed to generate a filter. For example, eye-blink switches can be utilized that detects changes in reflected infrared light that are a consequence of eyelid movements. In an aspect, kinematic analysis of eyelid movements for blink detection can be used to set temporal cutoffs that only respond to the reflectance changes associated with the intermediate temporal duration of the intentional blink/wink.
As another example, a characteristic of a sound can be determined and processed to generate a filter. A filter can be generated to discriminate between the sounds made by small tongue movements and ambient noise. Two modes of filtering can be used, specifically, a filter in the frequency domain and a filter in the time domain. For example, a candidate signal can be first passed through a band-pass filter to eliminate candidate signals that are not in a known frequency range of the intentional gesture sound. However, other environmental noises may occur in similar frequency ranges as the intentional gesture sounds, accordingly, a temporal filtering can also be applied. For example, background noises can be of random temporal durations, while the intentional gesture sounds can be of known temporal durations. Therefore, by ignoring candidate signals that fall within a desired frequency range of the intentional gesture sounds and distinguishing temporal durations longer or shorter than a predetermined duration associated with an intentional gesture sound, the amount of false positive intentional inputs can be further reduced.
At block **703,** one or more devices associated with the intentional input can be determined. For example, a microcontroller **404** can not only detect the presence of an intentional input but can also track a number of inputs (intentional and/or unintentional) produced in succession. For example, one input (a first intentional tap, a first intentional blink, a first intentional pressure bulb squeeze, or a first intentional small mouth sound) can be used to activate a nurse call, while two inputs (e.g., a second intentional tap, a second intentional blink, a second intentional pressure bulb squeeze, or a second intentional small mouth sound) can be used to control an additional device (e.g., a fan or light), and three inputs (e.g., a third intentional tap, a third intentional blink, a third intentional pressure bulb squeeze, or a third intentional small mouth sound) can control yet another additional device (e.g., a TV). In an aspect, this allows a user who can only produce a single gesture to directly control a number of devices.

In another aspect, a user can control one or more devices by a plurality of gestures. As an example, a sequence of gestures can be used to control a particular device. For example, an intentional tap followed by an intentional small mouth sound can control a television, while two intentional taps followed by an intentional small mouth sound can control a light. An intentional eye blink and an intentional small mouth sound can control a fan.

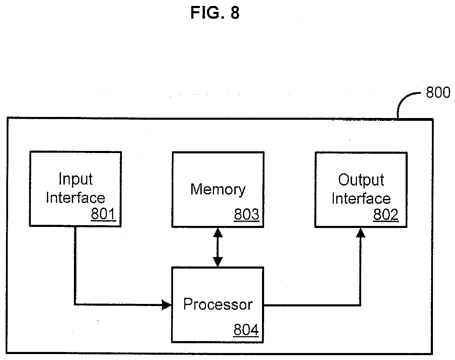

At block 704, one or more signals can be generated to control the one or more devices associated with the intentional input. As an example, controlling the one or more devices can comprise activating a nurse call switch. In another aspect, generating a signal in response to the intentional input can comprise controlling one or more controlled devices. A controlled device can comprise one or more of a nurse call switch, a computer, a television, a telephone, a medical device, an application (e.g., an iPad application), a speech generating device, and the like. In an aspect, the generated signal can be used as keyboard commands for communication or controlling other mechanical or computerized outputs. In an aspect, the one or more signals can comprise an optional visual output. The visual output can be displayed as a menu of instructions and options. As an example, the first signal can be generated to control a television, a second signal can be generated to control a light, and a third signal can be generated to control an air conditioning system.
In an aspect, illustrated in **FIG. 8****,** provided is an apparatus **800** for communication, comprising an input interface **801,** configured for receiving candidate input from one or more sensors, an output interface **802,** configured for transmitting a signal to a controlled device, a memory **803,** configured for storing a filter, and a processor **804,** coupled to the input interface **801,** the output interface **802,** and the memory **803.** In an aspect, the processor **804** can be configured to perform steps comprising, classifying the candidate input received by the input interface by applying the filter stored in the memory, and providing the signal to the output interface for transmission to the controlled device.

In an aspect, the input interface **801** can comprise one or more of a serial port, a Universal Serial Bus port, a Bluetooth transceiver, an 802.xx transceiver, and the like. The output interface **802** can comprise one or more of a serial port, a Universal Serial Bus port, a Bluetooth transceiver, an 802.xx transceiver, and the like. In a further aspect, the processor **804** can be further configured to perform steps comprising determining a characteristic of an intentional input and generating a filter based on the characteristic. The characteristic can derived from one or more of a frequency domain and a time domain.

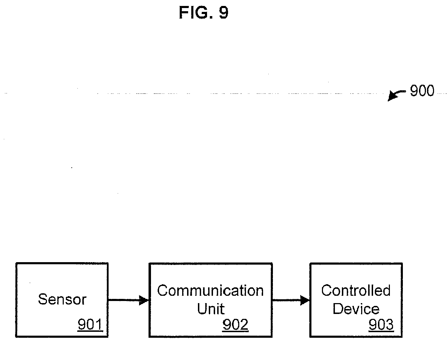

In an aspect, the memory **803** can stored filters and associated characteristics. When a candidate input is received, a filter can be retrieved from the memory **803** according to a characteristic of an intentional input. In an aspect, the characteristic can be derived from a frequency domain, a time domain, or combination thereof.
In an aspect, illustrated in **FIG. 9****,** provided are systems **900** for communication. The system **900** can comprise a sensor **901,** configured for receiving one or more candidate inputs, and a communication unit **902** coupled to the sensor **901.** In an aspect, the communication unit **902** can be configured to perform steps comprising, receive the one or more candidate inputs from the sensor **901,** classify the one or more candidate inputs as one or more intentional inputs, and generating a signal in response to the one or more intentional inputs. The system **900** can further comprise a controlled device **903** coupled to the communication unit **902.** In an aspect, the controlled device **903** can be configured to receive the signal generated by the communication unit **902.** The controlled device **903** can comprise one or more of a nurse call switch, a computer, a television, a telephone, a medical device, and the like. The sensor **901** can be one or more of a touch sensor, a pressure sensor, an infrared reflectance sensor, a light sensor, a mechanical sensor, a capacitance sensor, a proximity detector, an audio sensor, a camera, and the like.

In an aspect, the communication unit **902** can be further configured to perform steps comprising determining a characteristic of the one or more intentional inputs, generating a filter based on the characteristic, and applying the filter to the one or more candidate inputs. The characteristic can be derived from a frequency domain, a time domain, or combination thereof.

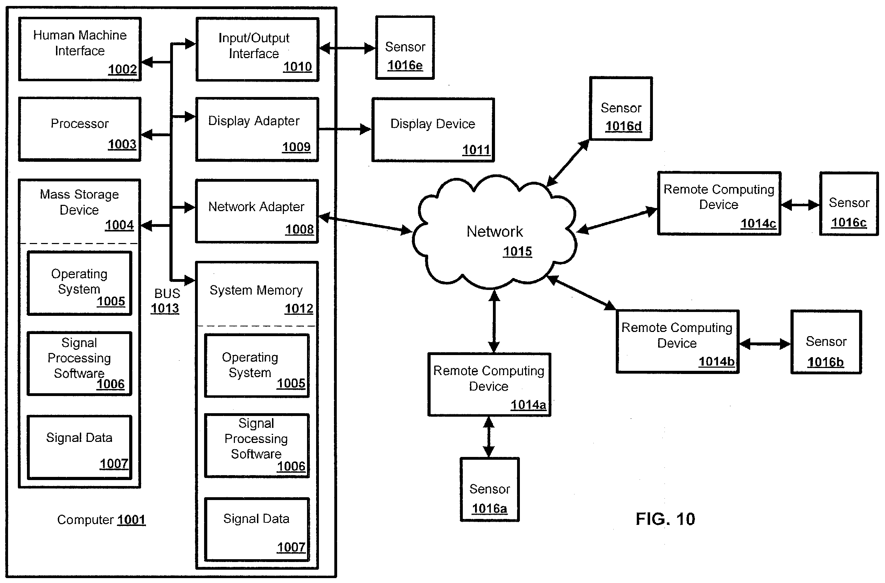

One skilled in the art will appreciate that provided is a functional description and that the respective functions can be performed by software, hardware, or a combination of software and hardware. The methods and systems can comprise the signal processing Software **1006** as illustrated in **FIG. 10** and described below. In one exemplary aspect, the methods and systems can comprise a computer **1001** as illustrated in **FIG. 10** and described below.

**FIG. 10** is a block diagram illustrating an exemplary operating environment for performing the disclosed methods. This exemplary operating environment is only an example of an operating environment and is not intended to suggest any limitation as to the scope of use or functionality of operating environment architecture. Neither should the operating environment be interpreted as having any dependency or requirement relating to any one or combination of components illustrated in the exemplary operating environment.

The present methods and systems can be operational with numerous other general purpose or special purpose computing system environments or configurations. Examples of well known computing systems, environments, and/or configurations that can be suitable for use with the systems and methods comprise, but are not limited to, personal computers, server computers, laptop devices, and multiprocessor systems. Additional examples comprise set top boxes, programmable consumer electronics, network PCs, minicomputers, mainframe computers, distributed computing environments that comprise any of the above systems or devices, and the like.

The processing of the disclosed methods and systems can be performed by software components. The disclosed systems and methods can be described in the general context of computer-executable instructions, such as program modules, being executed by one or more computers or other devices. Generally, program modules comprise computer code, routines, programs, objects, components, data structures, etc. that perform particular tasks or implement particular abstract data types. The disclosed methods can also be practiced in grid-based and distributed computing environments where tasks are performed by remote processing devices that are linked through a communications network. In a distributed computing environment, program modules can be located in both local and remote computer storage media including memory storage devices.

Further, one skilled in the art will appreciate that the systems and methods disclosed herein can be implemented via a general-purpose computing device in the form of a computer **1001.** The components of the computer **1001** can comprise, but are not limited to, one or more processors or processing units **1003,** a system memory **1012,** and a system bus **1013** that couples various system components including the processor **1003** to the system memory **1012.** In the case of multiple processing units **1003,** the system can utilize parallel computing.

The system bus **1013** represents one or more of several possible types of bus structures, including a memory bus or memory controller, a peripheral bus, an accelerated graphics port, and a processor or local bus using any of a variety of bus architectures. By way of example, such architectures can comprise an Industry Standard Architecture (ISA) bus, a Micro Channel Architecture (MCA) bus, an Enhanced ISA (EISA) bus, a Video Electronics Standards Association (VESA) local bus, an Accelerated Graphics Port (AGP) bus, and a Peripheral Component Interconnects (PCI), a PCI-Express bus, a Personal Computer Memory Card Industry Association (PCMCIA), Universal Serial Bus (USB) and the like. The bus **1013,** and all buses specified in this description can also be implemented over a wired or wireless network connection and each of the subsystems, including the processor **1003,** a mass storage device **1004,** an operating system **1005,** signal processing software **1006,** signal data **1007,** a network adapter **1008,** system memory **1012,** an Input/Output Interface **1010,** a display adapter **1009,** a display device **1011,** and a human machine interface **1002,** can be contained within one or more remote computing devices **1014a,b,c** at physically separate locations, connected through buses of this form, in effect implementing a fully distributed system.

The computer **1001** typically comprises a variety of computer readable media. Exemplary readable media can be any available media that is accessible by the computer **1001** and comprises, for example and not meant to be limiting, both volatile and non-volatile media, removable and non-removable media. The system memory **1012** comprises computer readable media in the form of volatile memory, such as random access memory (RAM), and/or non-volatile memory, such as read only memory (ROM). The system memory **1012** typically contains data such as signal data **1007** and/or program modules such as operating system **905** and signal processing software **1006** that are immediately accessible to and/or are presently operated on by the processing unit **1003.**

In another aspect, the computer **1001** can also comprise other removable/non-removable, volatile/non-volatile computer storage media. By way of example, **FIG. 10** illustrates a mass storage device **1004** which can provide non-volatile storage of computer code, computer readable instructions, data structures, program modules, and other data for the computer **1001.** For example and not meant to be limiting, a mass storage device **1004** can be a hard disk, a removable magnetic disk, a removable optical disk, magnetic cassettes or other magnetic storage devices, flash memory cards, CD-ROM, digital versatile disks (DVD) or other optical storage, random access memories (RAM), read only memories (ROM), electrically erasable programmable read-only memory (EEPROM), and the like.

Optionally, any number of program modules can be stored on the mass storage device **1004,** including by way of example, an operating system **1005** and signal processing software **1006.** Each of the operating system **1005** and signal processing software **1006** (or some combination thereof) can comprise elements of the programming and the signal processing software **1006.** Signal data **1007** can also be stored on the mass storage device **1004.** Signal data **1007** can be stored in any of one or more databases known in the art. Examples of such databases comprise, DB2®, Microsoft® Access, Microsoft® SQL Server, Oracle®, mySQL, PostgreSQL, and the like. The databases can be centralized or distributed across multiple systems.

In another aspect, the user can enter commands and information into the computer **1001** via an input device (not shown). Examples of such input devices comprise, but are not limited to, a keyboard, pointing device (*e.g.,* a "mouse"), a microphone, a joystick, a scanner, tactile input devices such as gloves, and other body coverings, and the like These and other input devices can be connected to the processing unit 1003 via a human machine interface **1002** that is coupled to the system bus **1013,** but can be connected by other interface and bus structures, such as a parallel port, game port, an IEEE 1394 Port (also known as a Firewire port), a serial port, or a universal serial bus (USB).

In yet another aspect, a display device **1011** can also be connected to the system bus **1013** via an interface, such as a display adapter **1009.** It is contemplated that the computer **1001** can have more than one display adapter **1009** and the computer **1001** can have more than one display device **1011.** For example, a display device can be a monitor, an LCD (Liquid Crystal Display), or a projector. In addition to the display device **1011,** other output peripheral devices can comprise components such as speakers (not shown) and a printer (not shown) which can be connected to the computer **1001** via Input/Output Interface **1010.** Any step and/or result of the methods can be output in any form to an output device. Such output can be any form of visual representation, including, but not limited to, textual, graphical, animation, audio, tactile, and the like. The display **1011** and computer **1001** can be part of one device, or separate devices.

One or more sensors **1016a-e** can be configured to provide input to computer **1001** through the input/output interface **1010** and/or the network adapter **1008.** The computer **1001** can receive inputs from sensors directly connected to the computer **1001** and through the network **1015.**

The computer **1001** can operate in a networked environment using logical connections to one or more remote computing devices **1014a,b,c.** By way of example, a remote computing device can be a personal computer, portable computer, smartphone, a server, a router, a network computer, a peer device or other common network node, and so on.

Logical connections between the computer **1001** and a remote computing device **1014a,b,c** can be made via a network **1015,** such as a local area network (LAN) and/or a general wide area network (WAN). Such network connections can be through a network adapter **1008.** A network adapter **1008** can be implemented in both wired and wireless environments. Such networking environments are conventional and commonplace in dwellings, offices, enterprise-wide computer networks, intranets, and the Internet.

For purposes of illustration, application programs and other executable program components such as the operating system **1005** are illustrated herein as discrete blocks, although it is recognized that such programs and components reside at various times in different storage components of the computing device **1001,** and are executed by the data processor(s) of the computer. An implementation of signal processing software **1006** can be stored on or transmitted across some form of computer readable media. Any of the disclosed methods can be performed by computer readable instructions embodied on computer readable media. Computer readable media can be any available media that can be accessed by a computer. By way of example and not meant to be limiting, computer readable media can comprise "computer storage media" and "communications media." "Computer storage media" comprise volatile and non-volatile, removable and non-removable media implemented in any methods or technology for storage of information such as computer readable instructions, data structures, program modules, or other data. Exemplary computer storage media comprises, but is not limited to, RAM, ROM, EEPROM, flash memory or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by a computer.

The methods and systems can employ Artificial Intelligence techniques such as machine learning and iterative learning. Examples of such techniques include, but are not limited to, expert systems, case based reasoning, Bayesian networks, behavior based AI, neural networks, fuzzy systems, evolutionary computation (e.g. genetic algorithms), swarm intelligence (e.g. ant algorithms), and hybrid intelligent systems (e.g. Expert inference rules generated through a neural network or production rules from statistical learning).

The following examples are put forth so as to provide those of ordinary skill in the art with a complete disclosure and description of how the devices and/or methods claimed herein are made and used, and are intended to be purely exemplary and are not intended to limit the scope of the methods and systems

### Examples

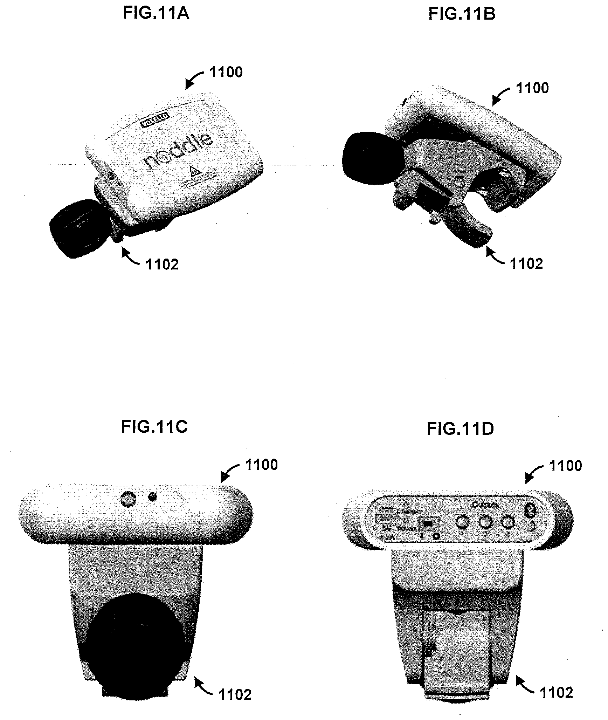

In an aspect, the present methods and systems can be implemented via an example apparatus illustrated in **FIG. 11** **-** **FIG. 21** and described in further detail herein. The apparatus can comprise the noddle™ developed by Voxello. The apparatus can be configured to specifically address the needs of patients who can not generate the movement or force necessary to use existing switch technologies. The apparatus can be configured for a user with limited physical abilities. For example, the apparatus can allow the user to utilize whatever type of sensor that is compatible with the user's physical capabilities. The apparatus can allow users who can only generate a single physical gesture to control multiple devices. This functionality is useful, for instance, for a user who can only blink one eye to be able to independently and simultaneously control a nurse call, television, fan and a speech generating device. The example apparatus can perform this functionality based on counting the number of times the user produces a gesture within a specified time window.

In an aspect, the apparatus can be configured to detect the smallest intentional gestures (e.g., tongue clicks, finger taps, eye blinks, applying minimal force to a low pressure sensing bulb). The apparatus can be configured to allow a patient, with a single switch, to control multiple devices, such as existing nurse call systems, speech-generating devices (SGD), patient controlled analgesia pumps, bed controls, telecommunication devices, environmental control systems, entertainment systems, and/or the like.

In an aspect, the apparatus can be configured to accept a range of sensors (e.g., noddle-mic™, noddle-touch™, and many 3^{rd} party switches) that can transduce whatever intentional gesture a patient can produce. The apparatus can be configured to use a gesture detection algorithm (e.g., as described herein) that filters out background noise and patients' unintentional gestures. Depending on whether a single gesture or sequence of gestures is detected, the apparatus can control at least three output devices. For example, the apparatus can communicate with the output devices via physical network links (e.g., serial or network communication) and/or wireless network links (e.g., via Bluetooth, WiFi, DEQ, Z-wave, or possibly new wireless technology in the future). For example, one intentional tongue click could activate the nurse call system, while two and three successive tongue clicks can be used to control a SGD (e.g., using the two-switch scanning mode). Therefore, an intensive care patient who cannot move or speak can use the apparatus to contact a nurse and also use a speech-generating device to communicate about pain or respiratory distress. Finally, the advantage of the apparatus over other "single" switches on the market is that patients would be able to take advantage of a "two-switch" scanning mode that allows them to enhance their communication rate by reducing the number of gestures needed to get to the desired message. In two-switch scanning the patient can move through a set of communication options by activating one switch and can then select to output an option by activating a second switch. For example, an output command (e.g., or signal) can be selected, and then the output device (e.g., or output port) can be selected. As another example, an output device (e.g., or output port) can be selected, and then an output command can be selected to output to the selected output device. The available commands for selection can be determined (e.g., or vary) based on the selected output device. The available output devices for selection can be determined (e.g., or vary) based on the selected output command.

**FIG. 11A** **-** **FIG. 11B** show multiple views of an example apparatus. **FIG. 11A** shows a top-down perspective view of the apparatus **1100** and a clamp **1102.** The clamp **1102** can comprise a pole clamp configured to mount the apparatus to a structure, such as a pole used for administering IV fluids. **FIG. 11B** shows a bottom-up perspective view of the apparatus **1100** and the clamp **1102.** **FIG. 11C** shows a side-view of the apparatus **1100** and the clamp **1102.** **FIG. 11D** shows another side-view of the apparatus **1100** and the clamp **1102.**

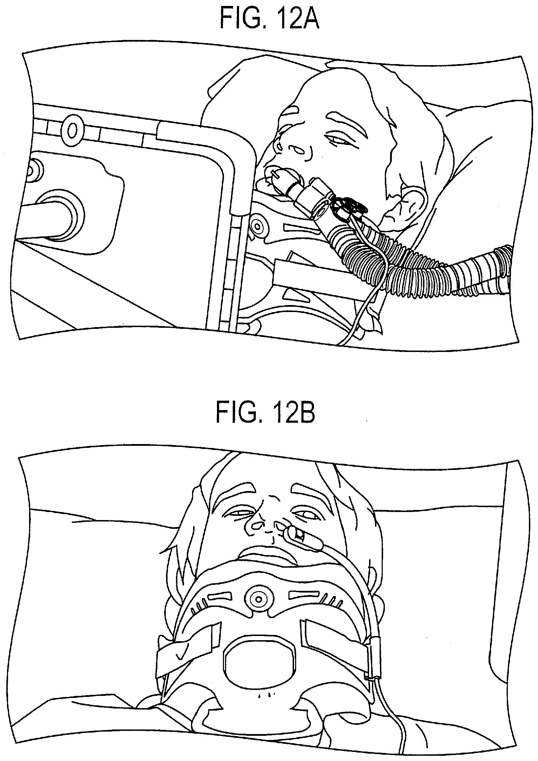

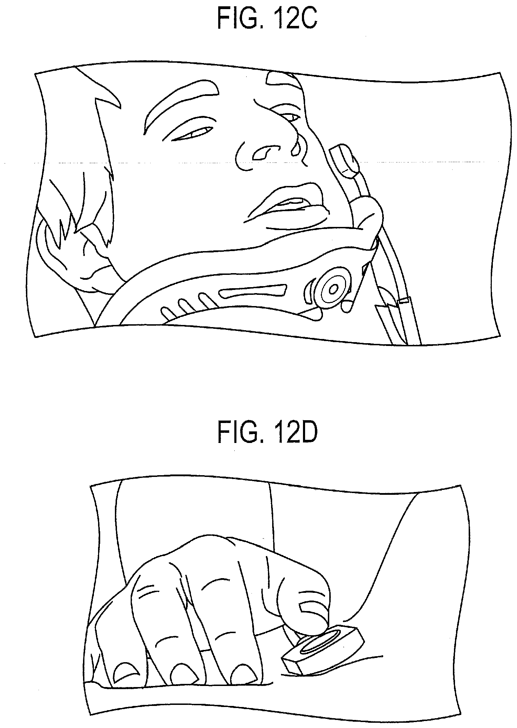

**FIG. 12A** **-** **FIG. 12D** illustrate some mounting options of sensors (e.g., noddle-mic™ and noddle-touch™ sensors) coupled with the apparatus. Example sensors can be mounted on a brace (e.g., a Miami-J collar), a vent line, bedding and/or the like. **FIG. 12A** shows a sensor mounted on a vent line. **FIG. 12B** shows a sensor mounted on a brace. **FIG. 12C** shows a sensor mounted on a brace. **FIG. 12D** shows a sensor mounted on bedding.

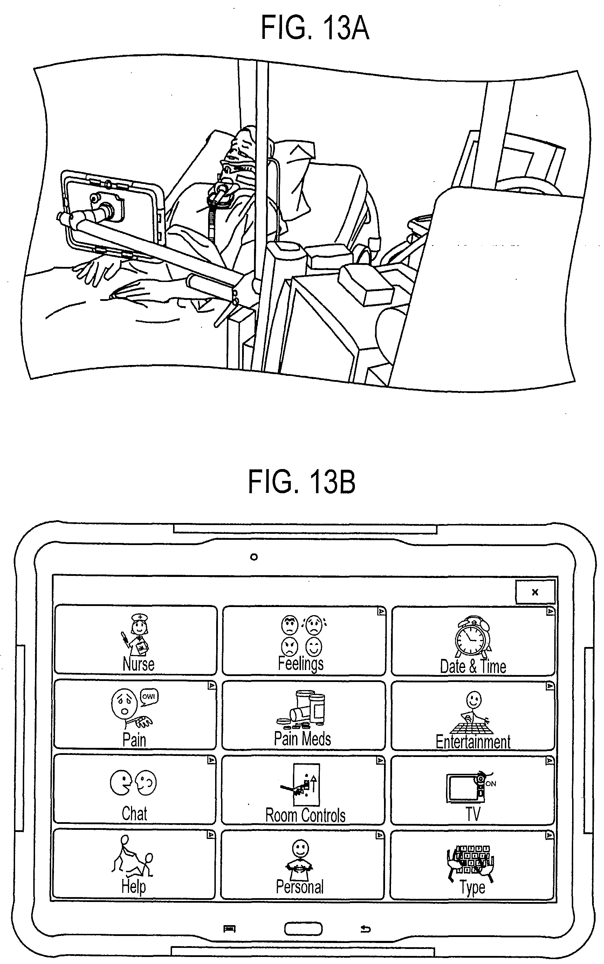

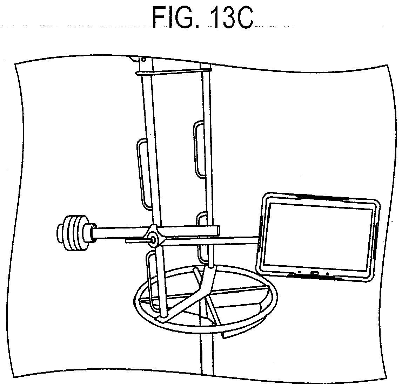

**FIG. 13A** illustrates an example apparatus with an IV pole mounted speech generating device (e.g., noddle-chat™) and customized mounting device (e.g., noddle-arm™) mounted on an IV pole. The mounting device allows a display to be provided to a user for easier control of multiple output devices. **FIG. 13B** illustrates an example user interface provided by the display. **FIG. 13C** shows another view of the mounting device and display device. The mounting device is attached to an IV pole via a clamp.

In an aspect, the apparatus can be used without calibration and can effectively be a "plug-and-play" device. The apparatus can be configured to automatically sense the type of sensor being used and apply the appropriate gesture detection algorithm. Since the apparatus is compatible with existing nurse call systems and speech-generating devices, the apparatus can be used in hospital and/or nursing home environments. The apparatus can be used with eye-tracking enabled SGDs. For example, patients with Amyotrophic Lateral Sclerosis (ALS), and other "locked in syndromes" can use the apparatus for communication. The primary obstacles to using eye trackers in acute care facilities involve calibrating the eye trackers, positioning the device at the bedside, and the added strain of having to maintain fixation to make a selection. Using the example apparatus would eliminate the added strain for patients who are able to use shift their gaze and use eye trackers.

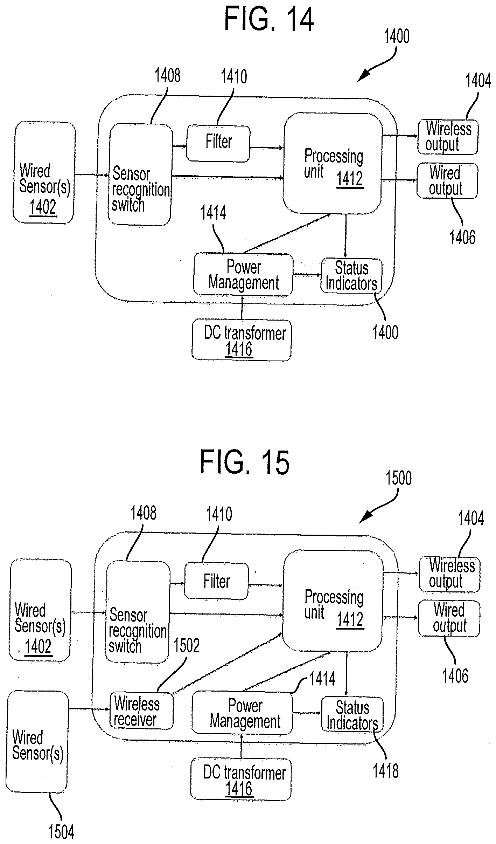

The following provides, in more detail, example technical specifications for the apparatus. **FIG. 14** is a block diagram illustrating an example apparatus **1400.** The apparatus **1400** can be configured to receive input from wired sensors **1402.** The apparatus **1400** can process that input and provide wired and/or wireless signals via a wireless output **1404** and/or a wired output **1406.**

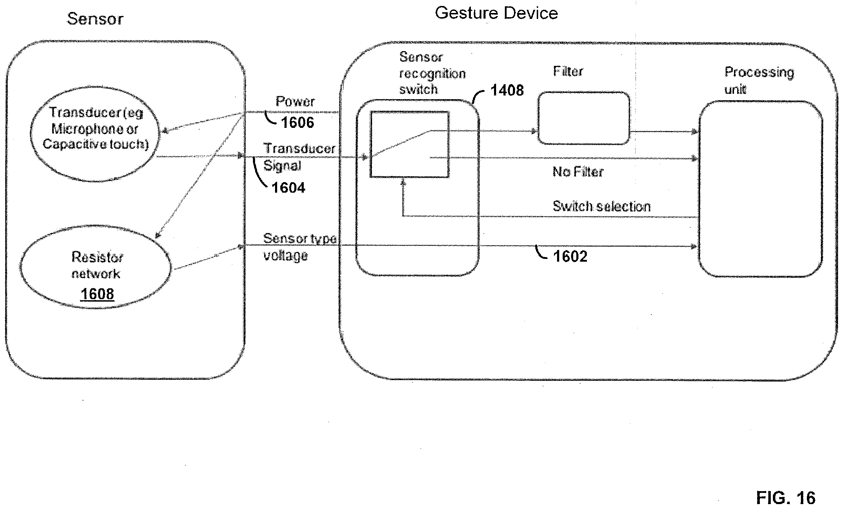

In an aspect, the apparatus **1400** can comprise a sensor recognition unit **1408** (e.g., or sensor recognition switch). As illustrated in more detail in **FIG. 16****,** The sensor recognition unit **1408** can be configured to determine a type of the wired sensor **1402.** The type of sensor (e.g., or information indicative of the type) can be provided to a processing unit **1412,** which can determine whether input signals are intentional, whether multiple signals are intended as a single gesture, and/or other information based on the type of the sensor. The sensor recognition unit **1408** can provide the input signal to a filter **1410.** The filter **1410** can filter out unintentional signals as described further herein. For example, the filter **1410** can filter (e.g., block, discard, ignore) repetitive signals above or below a frequency. The filter **1410** can filter signals that do not have expected characteristics (e.g., in the time domain or frequency domain). The sensor recognition unit **1408** can also provide input signals to the processing unit **1412.** In some implementations, the input signals can be provided to the filter **1410** and/or the processing unit **1410** without passing through the sensor.

The apparatus **1400** can be battery operated and/or receive power from other power sources. The apparatus **1400** can comprise a power management system **1414.** The power management system **1414** can be configured to deliver, manage, conserve and/or the like power throughout the apparatus **1400.** The apparatus **1400** can comprise a transformer **1416** electrically coupled to the power management system **1414.** For example, the transformer **1416** can comprise a direct current transformer configured to transform an alternating current source to a direct current. In an aspect, the apparatus **1400** can comprise a one or more status indicator systems **1418** as described further herein.

As explained further herein, the processing unit **1412** can be configured to manage one or more of the components of the apparatus **1400.** The processing unit **1412** can be configured to determine output signals and select output devices to receive output signals. For example, the processing unit **1412** can determine the output signals based on a number of input signals, frequency of input signals, signal characteristics (e.g., amplitude, frequency, voltage, pattern), which sensors the input signals are received from, time in between the input signals, time the input signals are received (e.g., and how such timing corresponds to information indicated and/or shown on a display), and/or the like.
**FIG. 15** is a block diagram illustrating another example apparatus **1500.** In an aspect, the apparatus **1500** can comprise one or more of the components of the apparatus **1400** of **FIG. 14****.** The apparatus **1500** can be configured to receive input from one or more wireless sensors. In this implementation, the apparatus **1500** can be configured to accept input from a wireless receiver **1502** communicatively coupled (e.g., paired) with one or more wireless sensors **1504.**

In an aspect, the apparatus **1500** can detect an intentional gesture via one of the sensors based on the correct identification of the sensor(s) being used with the apparatus **1500.** The apparatus **1500** can receive input from a single sensor or multiple sensors. For example, The apparatus **1500** can receive input (e.g., input signals) from an acoustic microphone (e.g., noddle-mic™), a proximity/capacitance sensor (e.g., noddle-touch™), a pressure sensor (e.g., noddle-bulb™), an IR reflectance sensor (e.g., noddle-wink™), and/or any other appropriate sensor.

As an illustration, the acoustic microphone can be configured to respond to small intentional tongue clicks that even intubated patients are able to make. The a proximity/capacitance sensor can function as a proximity switch (e.g., responds to a change in capacitance that results by some part of the users body coming in proximity to the sensor) and so can detect small low force movements. The pressure sensor can be activated by compression of a small bulb by the user. The IR reflectance sensor can be activated by an eye blink/wink or by any part of the users body that can alter the IR reflectance beam. Use of a camera and a recognition algorithm to detect head movement or some other gesture can also be used by converting a position change signal into a switch signal.

**FIG. 16** is a block diagram of an apparatus illustrating aspects of an example sensor recognition switch. In an aspect, the apparatus can be configured to automatically detect the type of sensor being used. As shown in **FIG. 16****,** the apparatus can comprise a sensor recognition switch **1408.** The sensor recognition switch **1408** can be configured to recognize a sensor and/or a type of the sensor. The sensor recognition switch **1408** can determine a specific sensor and/or type of sensor (e.g., coupled to the apparatus, providing signals to the apparatus, in range of the apparatus). For example, the sensor recognition switch **1408** can be configured to determine a unique signal voltage produced by a sensor and/or type of sensor. The sensor recognition switch **1408** can determine a resistance value in a parallel input line **1602** that is unique for each sensor. As explained further herein, an additional resistance value (e.g., unique to each sensor, unique to each type of sensor) can be built-in or added to the sensor that would indicate the gesture timing threshold to be used in the gesture detection algorithm.

In an aspect, the sensor recognition switch **1408** can be configured to detect gestures as a function of the user's physical state. For example, a user in a first physical state can make gestures with a first speed, frequency, force, and/or the like. A user in a second physical state can make gestures with a second speed, frequency, force, and/or the like. The sensor recognition switch **1408** can be configured to determine whether gestures are intentional based on which portion of a user is making the gesture, a characteristic (e.g., size, strength, mobility, movement range) of the portion of the user is making the gesture, and/or the like. For example, the sensor recognition switch **1408** can be configured to determine whether a gesture of a limb (e.g., arm, leg) is intentional based on a first force, frequency, speed, and/or the like. The sensor recognition switch **1408** can be configured to determine whether a gesture of an finger, eye, eyelid, and/or the like is intentional based on a second force, frequency, speed, and/or the like.

In an aspect, different types of sensor can be associated with different timing thresholds. For example, the sensors can have fixed characteristics (e.g., voltage, resistance). Thus, the characteristics can be associated with specific timing thresholds. As another example, the sensors can be modified by a user (e.g., via hardware and/or software setting). For example, a sensor can comprise a switch to select different resistance values. Accordingly, the user can set the timing threshold by adjusting the resistance value of the sensor. Where the sensor characteristic is fixed, the user can adjust the timing threshold by using a different sensor or sensor type. In some implementations, an interface of the apparatus can be accessed to adjust which characteristics are associated with which timing thresholds.

In an aspect, the sensor recognition switch **1408** can be configured to determine the type of sensor based on information from the sensor. The information can comprise a code. In the case of wireless sensors, a unique wireless code assigned to a particular type of sensor can be provided by the sensor to the apparatus. The unique code can be used to indicate, determine, and/or the like the gesture timing threshold for a specific sensor. A code can also be provided by wired sensor for identification of the sensor and/or type of sensor.

In an aspect, the sensor recognition switch **1408** can be configured to determine the type of sensor based on hardware and/or software characteristics associated with the sensor. For example, a sensor can be coupled to the apparatus via at least two input lines. A first input line **1604** can provide sensor output from a user. The second input line **1602** can be in parallel with the first input line **1602.** The second input line **1602** can comprise a resistor. The apparatus can determine the resistance, voltage drop and/or other characteristic indicative of the sensor based on the first input line **1604** and/or second input line **1602.** For example, a signal received from the second input line **1602** can be compared to a power output line **1606** (e.g., of the sensor, a power line from the apparatus to the sensor, a power line of a power source received by the apparatus and/or sensor) to determine the signal (e.g., or information, resistance, value) produced by the resistor network **1608.** A voltage difference or other difference in characteristics of the signals from the first input line **1604** and power output line **1606** can be determined to identify the sensor and/or type of sensor.

In another aspect, an input signal sensing algorithm can be used to determine the sensor and/or type of the sensor. For example, sensors and/or types of sensors can be associated with corresponding characteristics, such as signal voltages, signal patterns, signal frequencies, signal waveforms (e.g., sinusoidal, square, triangle), and/or the like.

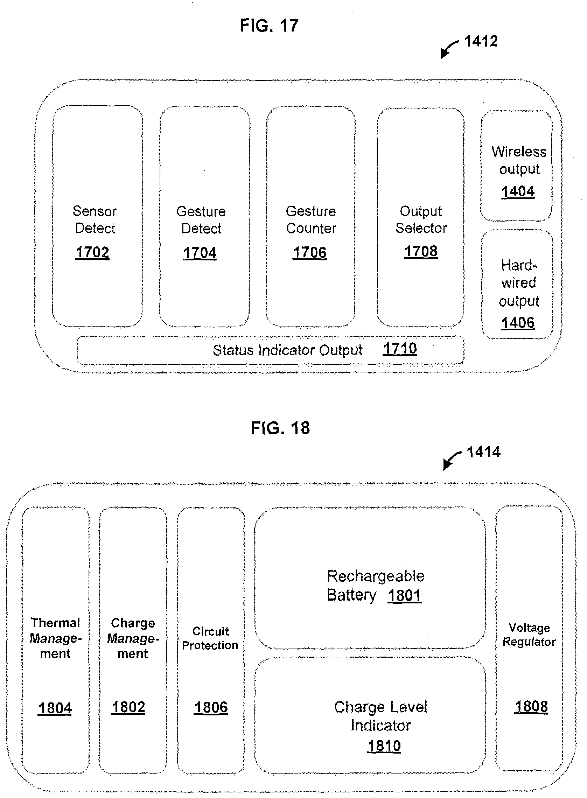

**FIG. 17** illustrates an example processing unit **1412** of the apparatus. The processing unit **1412** can comprise a sensor detection unit **1702.** The sensor detection unit **1702** can be configured to detect a sensor and/or type of sensor coupled (e.g., communicatively coupled) to the apparatus. For example, the sensor detection unit **1702** can comprise and/or manage the sensor recognition switch **1408.** The processing unit **1412** can comprise a gesture detection unit **1704** configured to classify input signals as intentional or unintentional gestures as explained herein. The processing unit **1412** can comprise a gesture counter unit **1706** configured to count gestures as explained herein. The processing unit **1412** can comprise an output selection unit **1708** configured to select an output based on the number of gestures counted by the gesture counter unit **1706.** The output selection unit **1708** can select one or more wireless outputs **1404** and/or wired outputs **1406.** The processing unit **1412** can also comprise a status indicator output configured to indicate the status of the processing unit **1412.**

When the apparatus is used with a single sensor, the apparatus can allow the user to effectively have the equivalent of at least three switches that can be linked to a range of devices. This is accomplished by the gesture counting algorithm. For example, the first detected gesture can initiate a counting window. The counting window can define an inter-gesture interval (IGI). For an additional gesture to be added to the count a minimal IGI must be exceeded. In addition, if a maximum IGI is exceeded, then the next signal received can be determined as a unique gesture rather than one of a series of related gestures (e.g., or a series of signals related to a signal gesture). An illustration of the gesture counting algorithm using a single sensor is as follows. If the maximum IGI is exceeded without additional detected gesture, then the apparatus will selected (e.g., set, determine) output 1. If a second gesture is detected within the IGI and then the maximum IGI is exceeded without an additional gesture, then the apparatus can selected output 2. If a third gesture is detected within the IGI and then the maximum IGI is exceeded without an additional gesture, then the apparatus can select output 3. If a fourth gesture is detected within the IGI, then the apparatus can determine not to select any output. This last option is included to allow the user to abort if he/she wishes to not have any output set.

**Table 1** illustrates an example relationship between the number of counted gestures and the output selected when only a single sensor is used.

**Table 1**

| Number of Gestures | Output Selected |
|---|---|
| 1 | 1 |
| 2 | 2 |
| 3 | 3 |
| 4 | (no output)- functions to abort sequence |

**Table 2** illustrates the relationship between the number of gestures counted and the output selected when more than one sensor is used. As an illustration, multiple sensors can be used to select outputs as follows. If the maximum IGI is exceeded without additional detected gesture and if the gesture was detected by sensor A, the apparatus can select output 1. If the maximum IGI is exceeded achieved without additional detected gesture and if the gesture was detected by sensor B, the apparatus can select (e.g., determine, set) output 2. If a second gesture is detected from the same sensor (e.g. sensor A) within the IGI and then the maximum IG is exceeded without an additional gesture, then the apparatus can select output 3. If a second gesture is detected from the same sensor (e.g. sensor B) within the IGI and then the maximum IGI is exceeded without an additional gesture, then the apparatus can select output 4. If a second gesture is detected from a different sensor (e.g. sensor A followed by sensor B) within the IGI and then the maximum IGI is exceeded without an additional gesture, then the apparatus can select output 5. If a second gesture is detected from a different sensor (e.g. sensor B followed by sensor A) within the IGI and then the maximum IGI is exceeded without an additional gesture, then the apparatus can select output 6. If three gestures are detected within the IGI, regardless of which sensors are activated, then the apparatus can select no output. Any three gesture sequence allows the user to abort if he/she wishes to not have any output set. It should be understood that the apparatus can be configured to count longer strings of gestures from one or more sensors thereby allowing for control of a larger number of outputs.

**Table 2**

| **First Gesture Detected Sensor ID** | **Second Gesture Detected Sensor ID** | **Third Gesture Detected Sensor ID** | **Output Selected** |
|---|---|---|---|
| A | - | - | 1 |
| B | - | - | 2 |
| A | A | - | 3 |
| B | B | - | 4 |
| A | B | - | 5 |
| B | A | - | 6 |
| A | A | A or B | (no output) |
| B | B | A or B | (no output) |
| A | B | A or B | (no output) |
| B | A | A or B | (no output) |

As illustrated in in **FIG. 14, FIG. 15****,** and **FIG. 17****,** the apparatus can provide both hardwired and wireless outputs. The hardwired outputs can be isolated relay outputs (e.g., an electrically operated switch). The isolated relay outputs can be configured as either normally open or normally closed. In an aspect, the apparatus can provide output signals comprising commands, characters (e.g., keyboard characters), function parameters, device settings. For example, the output can be in the form of a set (e.g., string, array, formatted data structure) of characters (e.g., keyboard characters). The apparatus also can provide the output via a serial port (e.g., USB port), wireless transceiver, network interface, and/or the like. Example commands can comprise a power on, power off, volume up, volume down, channel up, channel down, pause, play, fast forward, rewind, mute, open application, close application, like command, bookmark command, nurse call command, dial number, temperature setting, temperature up, temperature down, light on, light off, medication request, indicate an increase in pain level, indicate a decrease in pain level, changing feeling status, and/or the like. As another example, the command can comprise any language, speech, text, audio and/or the like generated by a user by issuing commands (e.g., selecting letters, words, or otherwise generating output) to a speech generation device. Through a speech generation device coupled to the apparatus there is almost no limit as to what the patient can communicate depending on how the device is programmed and set up.

In an aspect, the processing unit **1412** can be configured to control the status indicator system **1418** which provides the user feedback about the status of the device hardware, about the detection of intentional gestures, and/or other useful information. For example, the status indicator system **1418** can comprise a power indicator (e.g., LED power indicator). The power indicator can provide a steady illumination (e.g., green light) when the device is on. The status indicator system **1418** can comprise a charging indicator. The charging indicator can provide steady illumination when a battery of the apparatus is charging. The charging indicator can flash to indicate a battery charge error. When the charging indicator is not illuminated, the charge is complete and/or the apparatus is not charging the battery. The status indicator system **1418** can comprise a low battery indicator. The low battery indicator can flash (e.g., flash red) when the battery is low. A noise can also be emitted (e.g., pulsing beep). The status indicator system **1418** can comprise a sensor connection indicator. When a sensor is not coupled to the apparatus, a signal (e.g., red illumination) can be provided by the sensor connection indicator. The status indicator system **1418** can comprise a gesture detection indicator. The gesture detection indicator can provide a number of flashes (e.g., blue flashes) corresponding to a number of detected gestures. The status indicator system **1418** can comprise an output selection indicator. The output selection indicator can provide a number of flashes (e.g., green flashes) indicator a number of the selected output.

**FIG. 18** illustrates an example power management system **1414.** The apparatus can be powered from either the electrical mains using a DC power adapter (e.g., DC transformer **1416)** or secondarily by an internal rechargeable battery **1801.** The power management system **1414** can comprise a charge management system **1802** configured to prevent over charging of the internal battery **1801.** The power management system **1414** also can comprise safety features like a thermal cutoff element **1804** to preclude the possibility of overheating and the risks of fire. The power management system **1414** can also comprise a circuit protection element **1806,** a voltage regulator **1808,** a charge level indicator **1810,** and/or other elements related to power management.

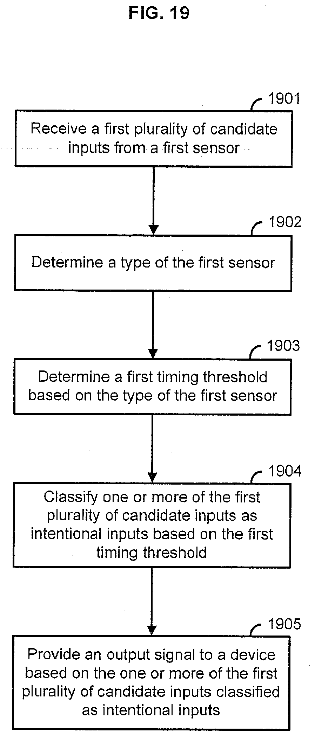

**FIG. 19** is a flow chart illustrating an example method **1900** for managing a device. At step **1901,** a first plurality of candidate inputs can be received from a first sensor. The first plurality of candidate inputs can comprise input signals. The first plurality of candidate input signals can be received via wired and/or wireless communication links.

At step 1902, a type of the first sensor can be determined. The type of the first sensor can be determined based on a voltage level of a signal received from the first sensor. In another aspect, determining the type of the first sense can comprise determining an electrical resistance value of a connection with the first sensor. The type of the first sensor can be determined based on the electrical resistance value.

At step **1903,** a first timing threshold can be determined based on the type of the first sensor. The first timing threshold can comprise a minimum duration of time between intentional inputs. For example, different types of sensors can be associated with different timing thresholds. The timing thresholds can be accessed in a data structure, database, and/or the like.

At step **1904,** one or more of the first plurality of candidate inputs can be classified as intentional inputs based on the first timing threshold. For example, a difference in time can be determined between a first time of receiving a first candidate input and a second time of receiving a candidate input prior (e.g., the immediately prior) to the first candidate input. If the difference is less than the first timing threshold, then the first candidate signal can be classified as unintentional. If the difference is greater than the first timing threshold, then the first candidate signal can be classified as intentional.

At step **1905,** an output signal can be provided to a device based on the one or more of the first plurality of candidate inputs classified as intentional inputs. The device can be selected based on a number of the first plurality of candidate inputs classified as intentional inputs. For example, step **1905** can comprise counting a number of the one or more of the first plurality of candidate inputs classified as intentional inputs. The device can be selected based on the number.

In another aspect, the method **1900** can further comprise receiving a second plurality of candidate inputs from a second sensor. A type of the second sensor can be determined. A second timing threshold can be determined based on the type of the second sensor. The device can be selected for receiving the output signal based on a combination of a number of the first plurality of inputs classified as intentional inputs and a number of the second plurality of inputs classified as intentional inputs.

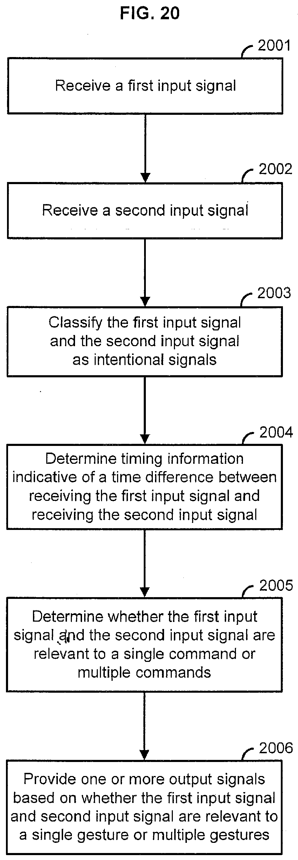

**FIG. 20** is a flow chart illustrating an example method **2000** for recognizing a gesture. At step **2001,** a first input signal can be received. The first input signal can be received from a first sensor. The first sensor can comprise touch sensor, a pressure sensor, a mechanical sensor, an optical reflectance sensor, an infrared reflectance sensor, a light sensor, a proximity detector, a capacitance sensor, an audio sensor, and a camera. As a further example, the first sensor can comprise an infrared reflectance sensor, a pressure bulb sensor, a proximity capacitance sensor, an acoustic microphone sensor, and/or any other appropriate sensor. The first sensor can be configured to provide only one type of signal or multiple types of signals (e.g., having variable voltages, frequencies, currents, and/or the like). For example, the first sensor can provide a single type of signal having a set characteristic (e.g., voltage, frequency, amplitude, pattern, and/or the like). In another aspect, the characteristics of the signal can vary based on the force applied by the user to the first sensor and/or other settings that the user can modify.

At step **2002,** a second input signal can be received after the first input signal. The second input signal can be received from the first sensor and/or a second sensor. The second sensor can comprise a touch sensor, a pressure sensor, a mechanical sensor, an optical reflectance sensor, an infrared reflectance sensor, a light sensor, a proximity detector, a capacitance sensor, an audio sensor, and a camera. As a further example, the second sensor can comprise an infrared reflectance sensor, a pressure bulb sensor, a proximity capacitance sensor, an acoustic microphone sensor, and/or any other appropriate sensor. The second sensor can be configured to provide only one type of signal or multiple types of signals (e.g., having variable voltages, frequencies, currents, and/or the like). For example, the second sensor can provide a single type of signal having a set characteristic (e.g., voltage, frequency, amplitude, pattern, and/or the like). In another aspect, the characteristics of the second signal can vary based on the force applied by the user to the second sensor and/or other settings that the user can modify. The characteristics of the second signal can be different than the characteristics of the first signal.

At step 2003, the first input signal and/or the second input signal can be classified as intentional signals. For example, the first input signal can be classified based on a first characteristic of the first input signal. The first characteristic can be a time domain characteristic, a frequency domain characteristic, and/or the like. For example, one or more filters (e.g., hi-pass filter, low pass filter) can be applied to the first input signal. The second input signal can be classified based on a second characteristic of the second input signal. The second characteristic can be a time domain characteristic, a frequency domain characteristic, and/or the like. For example, one or more filters (e.g., hi-pass filter, low pass filter) can be applied to the second input signal.

At step **2004,** timing information indicative of a time difference between receiving the first input signal and receiving the second input signal can be determined. For example, a first time indicative of the first input signal (e.g., a time when the first input signal is received) can be determined. A second time indicative of the second input signal can be determined (e.g., a time when the second input signal is received). The timing information can comprise a time difference between the first time and the second time (e.g., a length or duration of time between the first time and the second time.

At step **2005,** a determination can be made on whether the first input signal and the second input signal are relevant to a single command or multiple commands based on the timing information. For example, step **2005** can comprise comparing the timing information to a gesture time window. The gesture time window can comprise an maximum amount of time before two input signals (e.g., received in succession) are interpreted as relating to different gestures. The gesture time window can be specified by a user providing the first input signal and the second input signal. If the time difference is less than the gesture time window then the first input signal and the second input signal are determined to be relevant to a single gesture. If the time difference is greater than the gesture time window, then the first input signal and the second input signal can be determined to be relevant to two different gestures. In an aspect, the gesture time window can be based on a type of the first sensor and/or a type of the second sensor.

In another aspect, the gesture time window can initiated (e.g., start) in response to receiving the first input signal. The gesture time window can end after a set time. For example, if the end of the gesture time window is after the second time, then the first input signal and the second input signal can be determined to be relevant to the single gesture. If the end of the gesture time window is before the second time, then the first input signal and the second input signal can be determined to be relevant to two separate gestures.

At step **2006,** one or more output signals can be provided based on the determination of whether the first input signal and the second input signal are relevant to a single gesture or multiple gestures. For example, the device to provide the one or more output signals to can be selected based on a count of input signals received during the gesture time window.

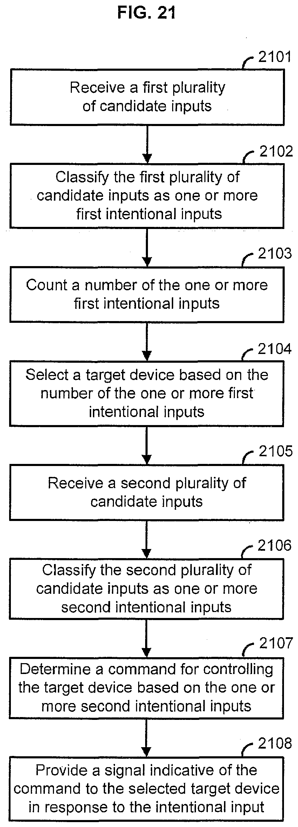

**FIG. 21** is a flow chart illustrating an example method **2100** for recognizing a gesture. At step **2101,** a first plurality of candidate inputs can be received. The first plurality of candidate inputs can be received from one or more sensors, via wired and/or wireless communication links. At step **2102,** the first plurality of candidate inputs can be classified as one or more first intentional inputs. For example, time domain and/or frequency domain characteristics of the first plurality of candidate inputs can be analyzed to determine which of the first plurality of candidate inputs are intentional as described further herein.

At step **2103,** a number of the one or more first intentional inputs can be counted. For example, a hardware or software counter can determine the number. The number can comprise a number counted during a gesture time window. At step **2104,** a target device can be selected based on the number of the one or more first intentional inputs. For example, the target device can be associated with the number. Each of a plurality of outputs can be give an a corresponding number.

At step **2105,** a second plurality of candidate inputs can be received. For example, after a target device is selected, a command detection time window can begin (e.g., be initiated). During the command detection time window, candidate inputs determined as intentional can be used to determine a command. At step **2106,** the second plurality of candidate inputs can be classified as one or more second intentional inputs. For example, time domain and/or frequency domain characteristics of the first plurality of candidate inputs can be analyzed to determine which of the first plurality of candidate inputs are intentional as described further herein.

At step 2107, a command for controlling the target device can be determined based on the one or more second intentional inputs. The command can comprise a command for an application on the target device. For example, the target device can be associated with and/or support a plurality of commands. The command can be determined based on a characteristic (e.g., a number, a pattern, a frequency, a voltage) of the one or more second intentional inputs. For example, the command can be associated with a corresponding number (e.g., or other characteristic) for the target device (e.g., or an application thereon). The number can be specific to the target device. As an illustration, if three intentional inputs are received in the command detection time window, then the command associated with the number three can be provided to the target device.

At step **2108,** a signal indicative of the command to the selected target device can be provided in response to the intentional input. The signal can comprise characters indicative of the command, voltages, signals patterns, amplitudes and/or other information for identifying the command.

Unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is no way intended that an order be inferred, in any respect. This holds for any possible non-express basis for interpretation, including: matters of logic with respect to arrangement of steps or operational flow; plain meaning derived from grammatical organization or punctuation; the number or type of embodiments described in the specification.

It will be apparent to those skilled in the art that various modifications and variations can be made without departing from the scope or spirit. Other embodiments will be apparent to those skilled in the art from consideration of the specification and practice disclosed herein. It is intended that the specification and examples be considered as exemplary only, with a true scope and spirit being indicated by the following claims.

## Claims

1. A method for communication, comprising:
detecting, by a computing device, a connection with a sensor configured to detect only a single gesture, wherein the computing device is configured to connect with a plurality of sensors of different types;
determining, by the computing device, a type of the sensor;
determining, by the computing device, a filter based on the type of the sensor, wherein the filter is generated based on analysis of signals associated with the type of sensor and configures the computing device to classify gestures as intentional or unintentional based on a first timing threshold and a frequency characteristic;
receiving, by the computing device from the sensor, a signal representing a plurality of candidate inputs detected by the sensor;
classifying, by the computing device, the plurality of candidate inputs as one or more intentional inputs based on the first timing threshold and the frequency characteristic;
determining, by the computing device, timing information indicative of a time difference between receiving a first input signal of the one or more intentional inputs and receiving a second input signal of the one or more intentional inputs;
determining, by the computing device, that the one or more intentional inputs are relevant to a single command based on the timing information;
counting, by the computing device, a number of the one or more intentional inputs relevant to the single command;
selecting, by the computing device, a target device from a plurality of devices, based on the number of the one or more intentional inputs relevant to the single command, wherein each of the plurality of devices is associated with a respective number and a number of the target device matches the number of the one or more intentional outputs relevant to the single command; and
providing a signal to the selected target device in response to the intentional input.

2. The method of claim 1 or 2, wherein the sensor comprises a touch sensor, a pressure sensor, a mechanical sensor, an optical reflectance sensor, an infrared reflectance sensor, a light sensor, a proximity detector, a capacitance sensor, an audio sensor, or a camera.

3. The method of any of claims 1-2, wherein providing the signal to the selected target device in response to the intentional input comprises activating one or more of a nurse call switch, a computer, a television, a fan, a light, a telephone, and a medical device.

4. The method of any of claims 1-3, wherein the type of the sensor is determined based on a voltage level of a signal received from the sensor, a code received from the sensor, an electrical resistance value of the connection with the sensor or a combination thereof.

5. The method of any of claims 1-4, further comprising:
receiving a second plurality of candidate inputs from the sensor;
classifying the second plurality of candidate inputs as one or more second intentional inputs; and
determining a command for controlling the target device based on the one or more second intentional inputs, wherein providing the signal to the selected target device comprises providing a signal indicative of the command to the selected target device in response to the intentional input.

6. The method of claim 5, wherein the command comprises a command for an application on the target device.

7. The method of any of claims 5-6, wherein the command is determined based on a number of the one or more second intentional inputs received during a time window, which target device is selected from among the plurality of devices, or a combination thereof.

8. A system, comprising:
a memory comprising computer-executable instructions; and
a processor functionally coupled to the memory and configured, by the computer-executable instructions, to perform at least the following actions,
receiving, from a sensor, a signal representing a plurality of candidate inputs detected by the sensor,
classifying the plurality of candidate inputs as one or more intentional inputs based on a filter,
determining timing information indicative of a time difference between receiving a first input signal of the one or more intentional inputs and receiving a second input signal of the one or more intentional inputs,
determining that the one or more intentional inputs are relevant to a single command based on the timing information,
counting a number of the one or more intentional inputs relevant to the single command,
selecting a target device from a plurality of devices based on the number of the one or more intentional inputs relevant to the single command, and
providing a signal to the selected target device in response to the intentional input.

9. The system of claim 8, wherein the sensor comprises a touch sensor, a pressure sensor, a mechanical sensor, an optical reflectance sensor, an infrared reflectance sensor, a light sensor, a proximity detector, a capacitance sensor, an audio sensor, or a camera.

10. The system of any of claims 8-9, wherein providing the signal to the selected target device in response to the intentional input comprises activating one or more of a nurse call switch, a computer, a television, a fan, a light, a telephone, and a medical device.

11. The system of any of claims 8-10, further comprising determining a type of the sensor, wherein the type of the sensor is determined based on a voltage level of a signal received from the sensor, a code received from the sensor, an electrical resistance value of the connection with the sensor, or a combination thereof.

12. The system of any of claims 8-11, wherein the processor is further configured for:
receiving a second plurality of candidate inputs from the sensor;
classifying the second plurality of candidate inputs as one or more second intentional inputs; and
determining a command for controlling the target device based on the one or more second intentional inputs, wherein providing the signal to the selected target device comprises providing a signal indicative of the command to the selected target device in response to the intentional input.

13. The system of claim 12, wherein the command comprises a command for an application on the target device.

14. The system of any of claims 12-13, wherein the command is determined based on a number of the one or more second intentional inputs received during a time window, which target device is selected from among the plurality of devices, or a combination thereof.

15. A non-transitory computer-readable medium comprising computer-executable instructions that when executed by a processor perform the method comprising:
receiving, by a computing device from a sensor, a signal representing a plurality of candidate inputs detected by the sensor;
classifying, by the computing device, the plurality of candidate inputs as one or more intentional inputs based on a filter;
determining, by the computing device, timing information indicative of a time difference between receiving a first input signal of the one or more intentional inputs and receiving a second input signal of the one or more intentional inputs;
determining, by the computing device, that the one or more intentional inputs are relevant to a single command based on the timing information;
counting, by the computing device, a number of the one or more intentional inputs relevant to the single command;
selecting, by the computing device, a target device from a plurality of devices, based on the number of the one or more intentional inputs relevant to the single command; and
providing a signal to the selected target device in response to the intentional input.
